# EUROPEAN PATENT APPLICATION

(11) **EP 4 275 582 A1**
(43) Date of publication of application: **15.11.2023**
(21) Application number: 23157973.1
(22) Date of filing: 22.02.2023
(51) Int. Cl.: A61B 1/00

(54) **ALBARRAN**

(30) Priority: 09.05.2022 US 202263339559 P
(71) Applicant: Olympus Winter & Ibe GmbH, 22045 Hamburg (DE)
(72) Inventor: Schmuck, Irina, 25746 Heide (DE); Schröder, Bastian, 22589 Hamburg (DE); Rühs, Andreas, 22926 Ahrensburg (DE); Schulz, Kevin Alexander, 22889 Wilstedt (DE)
(74) Representative: Hoener, Matthias

(57) **Abstract**

The invention provides an albarran that is particularly easy as well as thoroughly cleanable or reprocessable. This is achieved by connecting a proximal end (17) of a traction means outside a main body (11) of the albarran (10) to a traction unit (18). Through this traction unit (18), the traction means is translationally movable parallel to a shaft (12). The fact that the traction means is connected to the traction unit (18) outside the main body (11) means that this area, which was previously difficult to reach, can be cleaned very easily and thoroughly.

## Description

The invention relates to an albarran according to claim 1.

Albarrans are used as support for operations or treatments with surgical instruments, such as endoscopes, resectoscopes, cystoscopes or the like. An albarran can be used, for example, to deflect a flexible forceps within a patient in a targeted as well as controlled manner. For this purpose, the albarran, just like an endoscope for example, has a rod-like or tube-like shaft, which is to be guided into the patient's body with a distal end. At a proximal end of the albarran outside the patient, the shaft is connected to a main body. Via this main body, further instruments or tools, such as optics, wires or the like, can be guided through the shaft into the patient via various openings or ports.

At the distal end of the shaft, the albarran has a lever, the so-called albarran lever. This lever is designed to be movable and can be actuated or pivoted via a toggle on the main body. For this purpose, the lever is mechanically coupled to the toggle along the shaft. This mechanical coupling can be either a rod or a pull wire. As a rule, the lever is connected to the toggle via traction means, preferably with two pull wires. It is conceivable that both pull wires serve equally for pivoting the lever back and forth or that different movements of the lever via the toggle can be effected via the two pull wires.

For reliable operation of the albarran lever, the pull wires are to be tensioned inside the main body and there in a drive body or in a gear compartment with a pull wire driver. The pull wire driver is mounted on the shaft so that it can be moved along a longitudinal axis of the albarran. The rotational movement of the toggle is converted in the gear space into a linear movement of the pull-wire driver. This allows the toggle to be rotated to move the pull wire driver, and thus the wires, back and forth parallel to the shaft, rotating the lever at the distal end of the shaft about an axis. The gear is located in the gear chamber or drive body and is sealed to the outside by grease, adhesive, or gaskets. This sealing means that the gear compartment has no access or contact with the patient or user.

For reprocessing of the albarran or the main body, it is essential that all components, in particular internal components, can be rinsed, disinfected and sterilized. The sealing elements such as grease, adhesive and other seals age under various reprocessing processes (ultrasonic bath, machine cleaning, sterilization in autoclave). This can lead to leaks with potentially negative effects for reprocessability.

The invention is based on the problem of creating an albarran that can be cleaned and reprocessed particularly easily and thoroughly.

A solution to this problem is described by the features of claim 1. Accordingly, it is provided that a proximal end of a traction means is connected to a traction unit outside a main body of the albarran. By means of this traction unit, the traction means is translationally movable parallel to the shaft. Because the traction means is connected to the traction unit outside the main body, this previously difficult-to-reach area can be cleaned very easily and thoroughly. Since this end of the traction means is located outside the main body and also outside the shaft, it does not come into contact with liquids that come into contact with the albarran during the operation.

Preferably, the invention further provides that the traction unit comprises a carriage which is movable on the shaft and to which the proximal end of the traction means is couplable. In addition, the traction unit has an adjustment means for moving the carriage on the shaft. This traction unit also has an open design and does not have any encapsulations or cavities that would require increased effort to clean. Thus, the mechanism for moving the albarran lever is completely open and not enclosed by a housing as in known instruments. The proximal end of the traction means can be screwed or clamped to the carriage. The fact that the carriage is openly accessible makes it particularly easy to fix the traction means.

Preferably, the invention further provides that the lever and/or the carriage comprise a toothing, an eccentric or the like by which the lever and the carriage are movable relative to each other. For example, it is conceivable that the lever has an axle with a gear that engages a corresponding set of teeth on the carriage. Similarly, the lever could have an eccentric guide in which a pin of the carriage engages. Regardless of the nature of this translational mechanism, it is envisaged in accordance with the invention that the components of this mechanism are also exposed in order to ensure particularly easy as well as thorough cleaning of the albarran. By means of this mechanism, the operator can move the traction means parallel to the shaft during treatment and thereby operate the albarran lever.

A further advantageous embodiment of the invention can provide that the main body has at least one, in particular fluid-tight, seal through which the at least one traction means can be movably guided. This seal can be designed as a lead-through for the wire-like or rod-like traction means through the main body, wherein grease and/or a rubber seal can be used as sealing means. Through this seal, the traction means can be moved back and forth parallel to the shaft, while at the same time preventing fluid from escaping from the shaft to the outside. In this respect, any volume that can hold fluid associated with the treatment is limited to components that are supported distally in front of the main body. When viewed in the proximal direction, fluid can only collect inside the shaft or in further connections. This greatly reduces the amount of cleaning or reprocessing required. The seal in the main body used here can be replaced or the seal can be renewed after cleaning.

Furthermore, the invention can provide that the traction means is formed in one piece or in several pieces, whereby a multi-part traction means is composed of several traction means parts. The individual traction means parts can be connected to each other by coupling means. It is thus conceivable that one traction means part, viewed in the distal direction, is located in front of the main body and another is located behind the main body. The coupling means, which may be a plug-in, snap-in or screw connection, for example, may be part of the seal or is mounted in front of or behind the main body. One embodiment may provide that one traction means part is completely supported in the proximal area of the albarran and another traction means part is supported at least for the most part in the distal area. This separation allows for more efficient cleaning of the individual components, as the externally supported tensile member does not come into contact with fluid from the body.

Preferably, it is provided according to the invention that the at least one traction means and the at least one traction means part is formed as a pull wire and/or traction rod. For example, a rod could be attached directly to the carriage as a traction means, which is connected via a coupling means to another rod or to a wire in front of the main body as viewed in the distal direction. As already explained, the albarran lever can also be coupled to two pull wires or rods, which in turn are connected to the carriage by further wires or rods.

A preferred embodiment of the present invention is explained in more detail below with reference to the drawing. In this show:
- Fig. 1: a side view of an albarran,
- Fig. 2: a side view of a proximal end of the albarran according to Fig. 1, and
- Fig. 3: another side view of the proximal end of the albarran shown in Fig. 1.

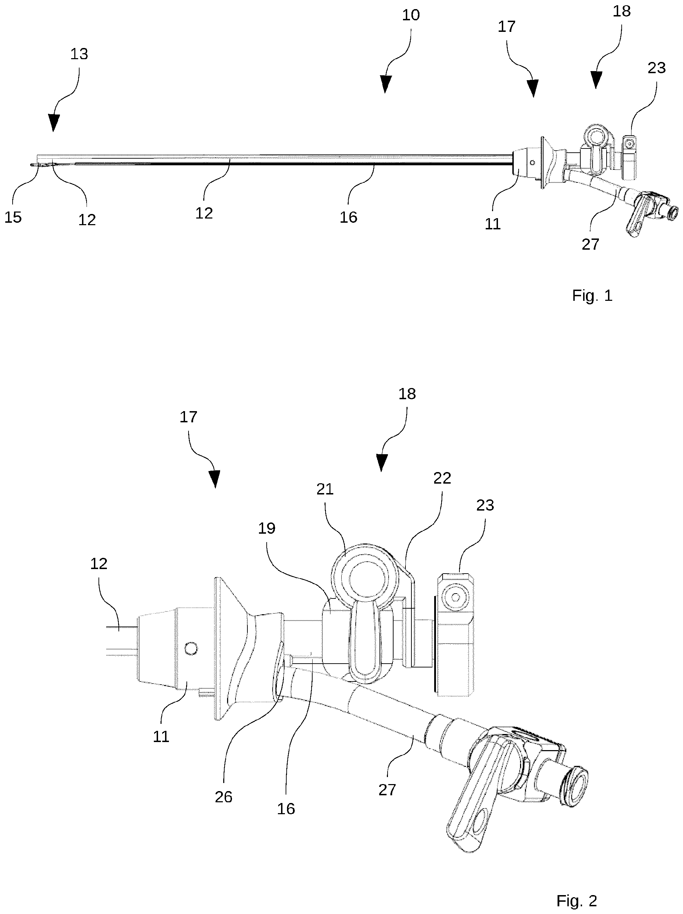

Fig. 1 shows a possible embodiment of an albarran 10. It should be expressly noted that the scope of protection of the present patent application is not intended to be limited to this embodiment. Rather, it is intended that further embodiments of the invention are also covered by the scope of protection.

The albarran 10 consists essentially of a main body 11 and a shaft 12 mounted on the main body 11. The shaft 12 is tubular and is inserted into the body of a person with a distal end 13 leading for the treatment of the person. Various medical or surgical instruments can be passed through the shaft 12. An albarran lever 15 is movably arranged on an outer wall 14 of the distal end 13 of the shaft 12. This lever 15 serves the surgeon during treatment as an aid for carrying out further treatment steps. The lever 15 is mounted so that it can be pivoted about an axis. To move the lever 15 about its axis, it can be actuated via pull wires 16. In addition, further connections 27 can be arranged on the main body 11 for the supply or removal of liquids or for the supply of further through instruments.

The pull wires 16 extend parallel to the shaft 12 from the lever 15 to the proximal end 17 of the albarran 10, where the pull wires 16 are fixedly connected to a traction unit 18. Via this traction unit 18, the pull wires 16 can be moved parallel to the shaft 12 so that the albarran lever 15 can be pivoted about its axis at the distal end 13 of the shaft 12. The traction unit 18 consists essentially of a carriage 19 and an adjusting means 20. As can be seen from Fig. 2, the carriage 19 is located on the shaft 12. For this purpose, the carriage 19 may, for example, have a bore through which the shaft 12 is guided. The pull wires 16 are attached to or in this carriage 19. Embodiments are conceivable in which the pull wires 16 are clamped, screwed, soldered or welded to the carriage 19. Embodiments are also known in which the lever 15 is connected and can be actuated by only one pull wire 16. Alternatively, it is also conceivable that the carriage 19 is connected to the lever by at least one rod or bar. A combination of a pull wire 16 with a rod is also one of the possible embodiments.

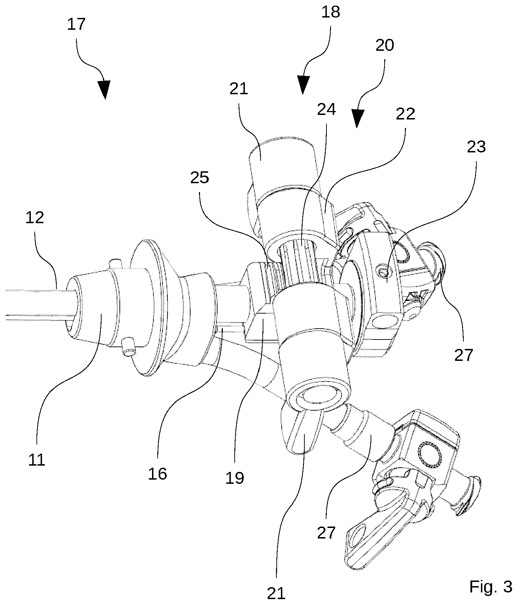

The translatory movement of the carriage 19 on the shaft 12 equally moves the at least one pull wire 16. The slide 19 in turn can be moved by the adjustment means 20. For this purpose, the adjusting means 20 is composed of a lever 21 and a holder 22. The lever 21 is rotatably attached to the holder 22 (Fig. 3). The axis of rotation of the lever 21 is aligned perpendicular to the longitudinal axis of the shaft 12. The holder 22 of the lever 21 is arranged either on the shaft 12 or on an optics plate 23 at the proximal end 17 of the albarran 10.

In the embodiment of the invention shown in Figs. 2 and 3, the longitudinal axis of the lever 21 has a gear wheel 24. This toothed wheel 24 engages in a toothing 25 on the carriage 19. By actuating the lever 21 or by rotating the lever 21 about its axis of rotation, the slide 21 is moved parallel to the longitudinal axis of the shaft 12 by the interaction of the gear 24 with the teeth 25. By actuating the lever 21, the operator can thus selectively move the albarran lever 15.

An essential feature of the invention is that the entire traction unit 18 with the carriage 19 and the adjustment means 20 is mounted outside the main body 11. Both the slide 19 and the adjustment means 20 are freely accessible without being encapsulated in any housings. This open design of the traction unit 18 makes both assembly and disassembly as well as cleaning particularly easy and thoroughly feasible. Since previous sealing of a corresponding housing was associated with considerable effort, particularly for cleaning, this open design of the traction unit 18 makes operation considerably easier.

In the embodiment example described here, only the pull wires 16 have to be guided through the main body 11. For this purpose, a seal 26 is provided inside the main body 11. This seal 26 prevents liquid from leaking out of the treatment area or the shaft area during treatment, or foreign bodies from entering the treatment area from the outside. This seal 26 can be, for example, a rubber seal, a grease layer or the like. It is also conceivable that a coupling is arranged inside the main body 11, in which a pull wire 16 or a pull rod engages from the outside and transmits force to a distally extending pull wire 16 or a pull rod.

### List of reference signs:

- 10: Albarran
- 11: Main body
- 12: Shaft
- 13: Distal end
- 14: Outer wall
- 15: Albarran Lever
- 16: Pull wire
- 17: Proximal end
- 18: Traction unit
- 19: Carriage
- 20: Adjustment means
- 21: Lever
- 22: Holder
- 23: Optical plate
- 24: Gear
- 25: Toothing
- 26: Seal
- 27: Connection

## Claims

1. Albarran (10) with a tubular shaft (12), at the distal end (13) of which an albarran lever (15) and at the proximal end (17) of which a main body (11) are arranged, the albarran lever (15) being movable via at least one traction means, **characterized in that** a proximal end (17) of the traction means is connected outside the main body (11) to a traction unit (18), by means of which the traction means can be moved translationally parallel to the shaft (12).

2. Albarran (10) according to claim 1, **characterized in that** the traction unit (18) comprises a carriage (19) movable on the shaft (12) and with which the proximal end (17) of the traction means is couplable, and an adjustment means (20) with which the carriage (19) is movable.

3. Albarran (10) according to claim 2, **characterized in that** the adjustment means (20) comprises a lever (21) rotatably mounted in a holder (22), the holder (22) being fixed to the shaft (12) or to an optics plate (23) at the proximal end (17) of the shaft (12).

4. Albarran (10) according to one of the preceding claims, **characterized in that** the lever (21) and/or the carriage (19) have a toothing (25) or an eccentric or the like, by means of which the lever (21) and the carriage (19) can be moved relative to one another.

5. Albarran (10) according to one of the preceding claims, **characterized in that** the traction means, in particular the carriage (19) and the adjusting means (20), is of open design, preferably the traction means (20) is not encapsulated or arranged in a housing.

6. Albarran (10) according to one of the preceding claims, **characterized in that** the main body (11) has at least one, in particular fluid-tight, seal (26) through which the at least one traction means can be movably guided.

7. Albarran (10) according to one of the preceding claims, **characterized in that** the tension means is formed in one part or in several parts, a multi-part tension means being composed of several tension means parts and the individual tension means parts being connectable to one another by coupling means.

8. Albarran according to claim 7, **characterized in that** the coupling means for the traction means parts is arranged in the seal (26) or outside the main body (11) at the proximal end (17) of the traction means.

9. Albarran (10) according to any one of the preceding claims, **characterized in that** a proximal portion of the at least one traction means and/or the at least one traction means part is exposed, in particular is not supported in a shaft (12) or a tube.

10. Albarran (10) according to one of the preceding claims, **characterized in that** the at least one traction means and the at least one traction means part is designed as a pull wire (16) and/or as a traction rod.
